# EUROPÄISCHE PATENTSCHRIFT

(11) **EP 2 587 098 B1**
(45) Veröffentlichungstag und Bekanntmachung des Hinweises auf die Patenterteilung: **13.08.2014**
(21) Anmeldenummer: 12185828.6
(22) Anmeldetag: 15.04.2009
(51) Int. Cl.: F16J 15/06

(54) **Vorrichtung zum Verdunsten von Flüssigkeit**
Device for evaporating liquids
Dispositif destiné à l'évaporation de liquides

(30) Priorität: 10.12.2008 DE 202008016286 U
(43) Veröffentlichungstag der Anmeldung: 01.05.2013
(62) Teilanmeldung aus: 09005524.5
(73) Patentinhaber: Meyer, Axel, 32805 Horn - Bad Meinberg (DE)
(72) Erfinder: Meyer, Axel, 32805 Horn - Bad Meinberg (DE)
(74) Vertreter: Emmerling, Friedrich

(56) Entgegenhaltungen:
- DE-A1- 10 103 155
- GB-A- 1 186 469
- JP-A- 10 250 755

## Beschreibung

### Gebiet der Erfindung

Die Erfindung betrifft eine Vorrichtung zum Verdunsten von Flüssigkeit mit kindersicherem Verschluss bzw. mit einem Verschluss, der das Öffnen der Vorrichtung im befüllten Zustand erschwert, sowie einen Verschluss hierfür.

### Stand der Technik

Die Literatur weist eine Vielzahl von Vorrichtungen auf, bei denen eine Flüssigkeit verdunstet und dabei ein Geruch abgegeben wird.

In der US 5,259,555 wird ein hölzerner, nachfüllbarer Behälter mit einer inneren Kammer für eine Duft abgebende Flüssigkeit beschrieben. In diese wird eine Duft abgebende Flüssigkeit gefiillt. Ein Plastikverschluss an der Unterseite des Behälters verhindert das Auslaufen der Flüssigkeit. Das im Behälter befindliche Öl durchdringt das Holz des Behälters und wird entlang der Kapillaren des Holzes an die Oberfläche des Behälters transportiert. An der Oberfläche verdunstet die Duft abgebende Flüssigkeit, wodurch der Duft so lange in die Umgebungsluft abgegeben wird, wie sich ausreichend Flüssigkeit im Behälter befindet.

Die DE 101 03 155 A1 beschreibt eine Verschlusskappe mit einer innenseitigen Dichteinrichtung zum flüssigkeitsdichten Verschließen eines Behälterhalses eines Behälters, wobei zwecks Dichtoptimierung die Dichteinrichtung mit einem Dichtelement aus quellbarem Material versehen ist für einen direkten oder indirekten kraftschlüssigen Kontakt mit dem oberen Behälterhalsbereich.

Die in der Druckschrift beschriebene Vorrichtung zum Verschließen des Behälters ist nur wenig dafiir geeignet, ein ungewolltes Öffnen, beispielsweise durch Kinder, zu verhindern.

Aufgabe der Erfindung ist es daher, eine Möglichkeit zu schaffen, bei einer Vorrichtung zur Verdunstung von Flüssigkeit ein unbefugtes bzw. unbeabsichtigtes Öffnen - insbesondere durch Kinder - und Ausfließen der Flüssigkeit zu verhindern.

### Lösung

Diese Aufgabe wird gemäß den unabhängigen Ansprüchen gelöst. Vorteilhafte Weiterbildungen sind Gegenstand der Unteransprüche.

Die Vorrichtung zum Verdunsten von Flüssigkeit weist einen Behälter zum Aufnehmen einer Flüssigkeit mit zumindest einer Behälteröffnung auf. Die Behälteröffnung kann mit einem Verschluss geschlossen werden, sodass die Flüssigkeit nicht auslaufen kann. Der Verschluss umfasst eine Dichteinrichtung, die zumindest teilweise aus einem Material gebildet ist, das durch Kontakt mit der Flüssigkeit aufquillt. Durch die damit verbundene Ausdehnung oder/und Formänderung wird die Behälteröffnung sicher verschlossen.

Die Formänderung des Materials, aus dem die Dichteinrichtung zumindest teilweise besteht, ist insbesondere reversibel oder zumindest zum Teil reversibel. Dadurch ist der Behälter, solange Kontakt zwischen der Flüssigkeit und dem Verschluss besteht, fest verschlossen und, wenn z. B. keine Flüssigkeit mehr vorhanden ist, wieder zu öffnen.

Bevorzugterweise sind die Dichteinrichtung und die Verdunstungseinrichtung so angeordnet, dass sie direkt oder indirekt in Kontakt mit der Flüssigkeit in dem Behälter stehen.

Durch das Quellen des Materials wird also eine wirksame Abdichtung realisiert, da, solange Flüssigkeit vorhanden ist, das Material aufgequollen und damit die Behälteröffnung fest verschlossen ist. Ist der Behälter jedoch entleert, so geht auch die mit der Quellung verbundene Formänderung des Verschlusses zurück, da er nicht mehr mit Flüssigkeit benetzt wird. Damit lässt sich der Verschluss wieder vom Behälter entfernen, nachdem der Behälter entleert ist. Bevorzugterweise lässt sich der Verschluss besonders gut wieder vom Behälter entfernen, wenn auch Reste der Flüssigkeit in der Verdunstungseinrichtung im Wesentlichen vollständig verdunstet sind. Eine Dichteinrichtung mit teilreversiblem, quellbarem Material weist dabei den Vorteil auf, dass die Vorrichtung auch im geleerten Zustand durch einen bestimmten Kraftschluss gegen unbeabsichtigtes Öffnen gesichert ist. Je nach Wahl des Verwendungszwecks der Vorrichtung, das heißt je nach Verwendung der Vorrichtung als wieder verwendbares oder als einmalverwendbares Produkt, wird das quellbare Material der Dichteinrichtung gewählt und damit bestimmt, wie leicht oder wie schwer sich der Behälter der Vorrichtung im entleerten Zustand öffnen lässt.

Dadurch wird mit einfachen Mitteln ein sicherer Verschluss bereitgestellt.

Bevorzugterweise handelt es sich bei dem Verschluss insbesondere um eine Verschlusskappe, die ganz oder teilweise aus einem Material besteht, das durch Kontakt mit der Flüssigkeit quillt. Durch das Quellen verändern sich das Volumen und damit die Ausmaße der Verschlusskappe, sodass die auf die Behälteröffnung aufgebrachte Verschlusskappe zumindest abschnittsweise enger an dieser anliegt als vor dem Quellen und so den Behälter flüssigkeitsdicht abschließt. Alternativ oder zusätzlich tritt durch das Quellen eine Ausdehnung bevorzugterweise in eine Richtung auf. Dann wird bei dieser Änderung die Fläche des Kraftschlusses vergrößert und somit der Kraftschluss verbessert. Es wird also durch das Quellen zumindest abschnittsweise eine kraftschlüssige Verbindung zwischen Behälteröffnung und Verschluss hergestellt oder verstärkt.

Teile der der Behälteröffnung zugewandte Innenseite des Verschlusses bildet bevorzugterweise dabei die Dichteinrichtung, sofern der Verschluss ganz oder teilweise aus einem Material besteht, das durch Kontakt mit der Flüssigkeit quillt. Alternativ ist die Dichteinrichtung an der der Behälteröffnung zugewandte Innenseite des Verschlusses angeordnet und besteht zumindest zum Teil aus quellbarem Material.

Entsprechend einer bevorzugten Ausführungsform der Erfindung ist der Verschluss einstückig mit der Dichteinrichtung und der Verdunstungseinrichtung ausgebildet und besteht im Wesentlichen aus quellbarem Material. Dabei steht zumindest ein Teil des Verschlusses direkt oder indirekt mit der in dem Behälter befindlichen Flüssigkeit in Kontakt. So wird die Flüssigkeit von dem quellbarem Material des Verschlusses aufgenommen, wobei zumindest der die Dichteinrichtung bildende Teil des Verschlusses aufquillt. Die von dem quellbarem Material des Verschlusses aufgenommene Flüssigkeit wird bevorzugterweise dabei auch an den die Verdunstungseinrichtung bildenden Teil des Verschlusses weitergegeben. Bevorzugterweise ist der die Verdunstungseinrichtung bildende Teil des Verschlusses zumindest ein Teil der Außenoberfläche des Verschlusses.

Bei einer alternativen Version der bevorzugten Ausführungsform der Erfindung verfügt der Verschluss zusätzlich über ein Element zum Aufnehmen der in dem Behälter befindlichen Flüssigkeit und Abgeben an die Dichteinrichtung und die Verdunstungseinrichtung des Verschlusses. Dies hat den Vorteil, dass konstruktiv mehr Möglichkeiten zur Verfügung stehen.

Der beim Quellen des Materials der Verschlusskappe entstehende Kraftschluss zwischen der Behälteröffnung, insbesondere einem Behälterhals, und der Verschlusskappe bewirkt, dass sich die Verschlusskappe nicht mehr leicht, insbesondere nicht mehr von Kinderhand, von der Behälteröffnung lösen lässt. Diese Wirkung verhindert ein unbefugtes oder unbeabsichtigtes Öffnen des Behälters und damit ein unerwünschtes Austreten der Duft abgebenden Flüssigkeit durch die Behälteröffnung. Damit wird ein Öffnen des Behälters durch Kinder verhindert und mit einfachen Mitteln eine Kindersicherung gewährleistet.

Bei dem Behälter kann es sich insbesondere um einen flaschenartigen Behälter oder Flakon handeln. Die Behälteröffnung ist insbesondere als ein Behälterhals ausgebildet, d. h. als ein im Wesentlichen röhrenförmiges Zwischenstück zwischen einem Bauch und dem Verschluss des Behälters. Das röhrenförmige Zwischenstück kann hierbei zumindest abschnittsweise zylindrisch oder kegelförmig oder konisch verlaufen, wobei die Form der Außenseite verschieden von der der Innenseite ausgebildet sein kann.

Ein Flakon ist ein meist aus Glas bestehendes Gefäß mit schmalem Hals und einem Bauch mit verschiedener Formgebung, beispielsweise rund, eckig etc. Der Behälter nimmt bevorzugterweise einen flüssigen und flüchtigen Duftstoff auf.

Bei der Verwendung einer bei Verdunstung einen Geruch abgebenden Substanz kann die Vorrichtung zur Duftabgabe eingesetzt werden. Diese Duft abgebende Flüssigkeit, z. B. ein ätherisches Öl - mit oder ohne Alkohol -, Parfüm etc., kann im Bauch des Behälters, insbesondere eines Flakons, aufbewahrt werden.

Der Flakon weist eine Einfüllöffnung auf, die als Hals ausgestaltet ist. Der Hals eines Flakons ist in der Regel derart gestaltet, dass er als röhrenförmiges Zwischenstück zwischen Bauch und Verschluss fungiert und auf diese Weise das Austreten der Duft abgebenden Flüssigkeit aus dem Flakon über eine Öffnung ermöglicht, die sich auf der dem Bauch abgewandten Seite des Halses befindet.

Gemäß einer vorteilhaften Ausgestaltung ist das Material, das der Verschluss zumindest teilweise umfasst, derart beschaffen, dass die Flüssigkeit durch das Material transportiert werden kann. Dadurch kann die Flüssigkeit zu einer äußeren Oberfläche, d. h. nicht der dem Inneren des Behälters zugewandten Oberfläche, transportiert werden und dort verdunsten.

Dies hat den Vorteil, dass das Material zugleich als Verdunstungseinrichtung eingesetzt werden kann, was einerseits die Herstellung vereinfachen kann und andererseits eine kompaktere Ausführung der Vorrichtung ermöglicht. Somit fungiert die außen liegende Oberfläche des Materials als Verdunstungseinrichtung.

Die Dichteinrichtung, also die Teile des Verschlusses, die in Kontakt mit der Behälteröffnung sind, wird gemäß einer vorteilhaften Ausgestaltung durch zumindest einen Vorsprung gebildet, der an der Seite des Verschlusses angeordnet ist, die, wenn der Verschluss auf die Behälteröffnung gesetzt ist, zum Behälter hingerichtet ist.

Gemäß einer Ausführungsform liegt damit der Vorsprung an der Innenseite, d. h. an der zum Behälterinneren gerichteten Seite, oder an der Außenseite der Behälteröffnung nach einem Aufquellen kraftschlüssig an. Gemäß einer anderen Ausführungsform ist der Kraftschluss zusätzlich oder alternativ auf der Oberseite der Behälteröffnung vorgesehen. Dies ermöglicht, dass weitgehend verhindert wird, dass überhaupt Flüssigkeit austritt.

Gemäß einer weiteren Ausführungsform sind mehrere, zumindest aber zwei, Vorsprünge vorgesehen, sodass zumindest je ein Vorsprung an der Innen- und der Außenseite der Behälteröffnung kraftschlüssig anliegt. Dies hat den Vorteil einer besonders guten Abdichtung der Behälteröffnung. Weiterhin wird durch das Anliegen zu beiden Seiten der Behälteröffnung ein Verziehen der Form des Verschlusses unterbunden oder zumindest reduziert, sodass der Verschluss über zumindest mehrere Quellvorgänge verwendbar ist.

Gemäß einer weiteren Ausgestaltung ist der zumindest eine Vorsprung korkenförmig ausgebildet, sodass er an der Innenseite des Behälterhalses anliegt. Damit wird ein besonders einfacher Verschluss realisiert. Der Korken ist vorzugsweise zylinder- oder konusförmig ausgebildet oder/und der Form der Innenseite der Behälteröffnung angepasst.

Gemäß einer weiteren vorteilhaften Ausgestaltung weist der zumindest eine Vorsprung eine Riffelung auf, die zumindest teilweise mit einer Riffelung an der Behälteröffnung korrespondiert. Dies erhöht weiterhin die erforderliche Kraft, um den Verschluss zu entfernen, und erhöht somit die Sicherheit, dass Kinder den Verschluss unbefugt öffnen. Bei einer Riffelung handelt es sich insbesondere um zumindest eine Erhöhung in einer Oberfläche gegenüber der Umgebung. Eine Riffelung kann also beispielsweise durch eine gewindeartige Struktur oder durch Noppen oder Rillen gebildet werden.

Gemäß einer weiteren Ausführungsform verbreitert sich die im Verschluss eingefügte Rille zu ihrem Boden hin. Dadurch wird die Klemmwirkung der zu beiden Seiten der Rille gebildeten Vorsprünge erhöht. Alternativ kann sich die Rille zum Boden hin verjüngen, sodass ein Aufsetzen des Verschlusses auf den Behälter erleichtert wird.

Gemäß einem Ausführungsbeispiel wird die Rille durch eine, insbesondere ringförmige, Fräsung gebildet, die mit der Form der Oberseite der Behälteröffnung korrespondiert. Insbesondere nimmt dabei die Dicke des Rings der ringförmigen Fräsung zum Inneren des Verschlusses hin ab. Dadurch lässt sich der Verschluss leichter auf die Behälteröffnung aufsetzen. Gleichzeitig kommt es dabei durch Verklemmen auch zu einem besseren Kraftschluss zwischen Verschluss und Behälteröffnung.

Eine andere Ausgestaltung sieht vor, dass sich alternativ oder zusätzlich der Innendurchmesser der Behälteröffnung zu der Außenseite des Behälters hin verjüngt. Auch dadurch kann die Klemmwirkung erhöht werden. Diese Verjüngung kann konstruktiv beispielsweise durch eine nach innen ragende Wulst an der Behälteröffnung realisiert werden. Auch eine konische Verjüngung des Innendurchmessers der als Flaschenhals ausgebildeten Behälteröffnung ist möglich. Beide Varianten bewirken, dass der Kraftschluss zwischen dem Verschluss und der Behälteröffnung durch einen beim Quellvorgang entstehenden Formschluss im Bereich der Wulst bzw. des geringeren Durchmessers an der äußeren Öffnung des Halses ergänzt wird. Ein Öffnen des Behälters ist damit nur mit sehr großer Kraft möglich, solange noch Flüssigkeit im Behälter ist. Ein sicheres Verschließen wird also insbesondere durch einen Kraft- oder/und Formschluss gewährleistet.

In einer weiteren Ausgestaltung ist die Dichteinrichtung einstückig mit dem Verschluss gebildet. Bevorzugterweise besteht der Verschluss dabei im Wesentlichen aus dem quellbaren Material oder ist beispielsweise mittels Mehrkomponentenspritzgießen zwar einstückig geformt, jedoch aus mehreren Materialien gefertigt. Dadurch wird eine besonders einfache Herstellung ermöglicht. Weiterhin ist durch die einstückige Ausführung eine hohe Stabilität gewährleistet.

Gemäß einer anderen Ausgestaltung umfasst die Vorrichtung weiterhin ein langgestrecktes Element, das bevorzugterweise an dem Verschluss befestigt ist und durch die Behälteröffnung ragt. Insbesondere ragt dieses langgestreckte Element im Wesentlichen bis zum Boden des Behälters. Durch dieses langgestreckte Element kann Flüssigkeit aus dem Behälter transportiert werden, bis dieser im Wesentlichen entleert ist. Bei dem langgestreckten Element handelt es sich insbesondere um einen Stab oder Docht oder Tampon oder Kombinationen aus diesen, der zum Transportieren der Flüssigkeit zu der Dichteinrichtung und/oder der Verdunstungseinrichtung an dem Verschluss angeordnet ist. Der Transport in dem langgestreckten Element erfolgt beispielsweise mittels Kapillarkräften, sodass die Flüssigkeit aufwärts befördert werden kann.

Als günstig hat es sich auch erwiesen, wenn der Stab aus Holz und insbesondere aus Peddigrohr bzw. Rattan besteht. Peddigrohr wird aus dem Stamm von Rotangpalmen gewonnen. Es zeichnet sich durch eine stark poröse Struktur aus, die dem Material eine geringe Dichte und besondere Elastizität verleiht. Diese Struktur bewirkt sehr gute Transporteigenschaften mittels Kapillarkräften.

Gemäß einer vorteilhaften Ausgestaltung handelt es sich bei dem Material, das der Verschluss zumindest teilweise umfasst, um Holz oder einen quellbaren Kunststoff.

Holz weist die Eigenschaft auf, dass es in Kontakt mit Flüssigkeit quillt und zudem Kapillaren aufweist, in denen Flüssigkeit transportiert werden kann. Als besonders geeignet hat es sich erwiesen, für die Herstellung des Verschlusses Erlenholz zu verwenden. Die Erle gehört zur Gattung der Laubgehölze. Sie zeichnet sich insbesondere durch günstige Transporteigenschaften in den Kapillaren aus, sodass eine Verdunstung mit einer Rate erfolgt, die z. B. für eine Duftabgabe bei Verdunstung günstig ist. Bei entsprechender Dimensionierung der Verdunstungseinrichtung kann somit z.B. erreicht werden, dass bei Verwendung von Erlenholz eine Flüssigkeitsmenge von ca. 30 ml im Verlauf von ca. 4 Wochen verdunstet.

Ähnlich geeignet sind Birkenholz und Pappelholz. Bei Verwendung von Buchenholz verdampft die Duft abgebende Flüssigkeit langsamer, da diese Holzart die Flüssigkeit langsamer transportiert. Buchenholz wird somit bevorzugterweise eingesetzt, wenn z.B. eine niedrige Verdunstungsrate erzielt werden soll. Bei Verwendung von Fichtenholz ist der Transport der Flüssigkeit deutlich höher als bei Buchenholz. Der Duft verströmt damit wesentlich schneller.

Demgegenüber haben andere Hölzer diesbezüglich ungünstigere Eigenschaften. Buche ist wegen der sehr geringen Kapillarwirkung überhaut nicht geeignet, da kaum Flüssigkeit transportiert wird, während bei Fichtenholz der Transport der Duft abgebenden Flüssigkeit relativ schnell erfolgt und somit die Zeitdauer der Duftabgabe zu kurz oder/und ggf. die Intensität des so verströmten Dufts zu hoch ist.

Gemäß einer Ausführungsform weist die Vorrichtung einen hölzernen Verschluss auf, wobei das Holz des Verschlusses derart gewählt ist, dass es von dem flüssigen Duftstoff zum Quellen gebracht werden kann. Das Holz des Verschlusses ist ferner derart gewählt, dass es den Duftstoff in Kapillaren im Holz transportieren kann.

Gemäß einer Ausführungsform weist der hölzerne Verschluss zur Aufnahme des Stabs eine zentrale Bohrung auf. Außerdem weist der hölzerne Verschluss eine ringförmige Fräsung um die zentrale Bohrung herum auf, die derart gestaltet ist, dass ein hölzerner Ring um die zentrale Bohrung erhalten bleibt. Die ringförmige Fräsung ist derart ausgebildet, dass der hölzerne Verschluss mit der ringförmigen Fräsung auf den Hals des Flakons aufgesetzt werden kann. Die Länge des Stabs ist dabei derart gewählt, dass er vom hölzernen Verschluss bis zum flüssigen Duftstoff reicht. Anstellte einer ringförmigen Fräsung können auch andersförmige Fräsungen gewählt werden, die an die Form der Behälteröffnung angepasst sind, beispielsweise ovale oder viereckige Fräsungen.

Bei einer weiteren Ausführungsform erfolgt das Verschließen der Öffnung des Halses durch Aufsetzen des hölzernen Verschlusses auf die Öffnung des Halses des Flakons. Der hölzerne Verschluss wird beispielsweise dem Flakon beiliegend mitgeliefert, kann aber auch bereits aufgesetzt sein. Im ersteren Fall ist der Flakon vor dem Aufsetzen des hölzernen Verschlusses durch einen Kindersicherungsverschluss aus Kunststoff verschlossen. Sobald der hölzerne Verschluss mit dem hölzernen Stab auf den Flakon aufgesetzt wird, taucht der Stab in die Duft abgebende Flüssigkeit ein. Diese wird durch die Kapillaren im Holz des Stabs zum hölzernen Verschluss transportiert. Durch die Kapillaren des hölzernen Verschlusses wird die Duft abgebende Flüssigkeit an die äußere Oberfläche des Verschlusses transportiert. Diese wird sichtbar durch die Duft abgebende Flüssigkeit benetzt. An der Oberfläche des hölzernen Verschlusses verdampft die Duft abgebende Flüssigkeit und gibt damit den Duft an die Umgebungsluft ab.

Gemäß einer anderen vorteilhaften Ausgestaltung ist das langgestreckte Element in eine Bohrung im Verschluss einsetzbar. Bei einem hölzernen Verschluss liegt diese Bohrung in der Ebene der Maserung des Holzes des Verschlusses und damit senkrecht zur Richtung der ehemaligen Baumspitze, von der das Holz stammt. Damit lassen sich weitere Verbesserungen der Transporteigenschaften und damit im Falle von Duftstoffen in der Flüssigkeit eine Duftabgabe mittels der Verdunstungseinrichtung erreichen.

Gemäß einer weiteren Ausgestaltung ist es vorgesehen, dass die Verdunstungseinrichtung mehrstückig ausgebildet ist und sich zumindest ein zusätzliches Element der Verdunstungseinrichtung am Verschluss befestigen lässt. Dieses zusätzliche Element der Verdunstungseinrichtung ist derart an den Verschluss gekoppelt, dass die Flüssigkeit in dieses zusätzliche Element der Verdunstungseinrichtung mittels des quellbaren Materials oder mittels eines anderen, die Flüssigkeit transportierenden Materials des Verschlusses transportiert werden kann. Dadurch lässt sich die Verdunstungsoberfläche weitgehend beliebig gestalten, insbesondere vergrößern, sodass eine gewünschte Verdunstungsrate erzielt werden kann.

Gemäß einer weiteren Ausgestaltung weist der Verschluss zumindest eine ebene Außenfläche auf. Dadurch kann erreicht werden, dass die gesamte Vorrichtung auf die ebene Fläche des Verschlusses, also beispielsweise auf den Kopf, gestellt werden kann. Die Flüssigkeit benetzt dabei bevorzugterweise das quellbare Material des Verschlusses direkt und nicht indirekt über ein zusätzliches Element, wie etwa ein Stab oder Docht. In einer vorteilhaften Ausführungsform wird weiterhin die ebene Fläche mit einer flüssigkeitsundurchlässigen Schicht, beispielsweise einer Lackschicht, versehen, sodass die Auflagefläche, auf der diese ebene Fläche aufliegt, beispielsweise eine Tischplatte oder ein Regal, nicht durch die Verdunstung oder austretende Flüssigkeit beeinträchtigt wird. Gemäß einer anderen Ausführungsform umfasst die Vorrichtung weiterhin einen Untersetzer auf den die ebene Fläche gestellt wird.

Die bei dieser weiteren Ausgestaltung mit der Flüssigkeit in direktem Kontakt stehende Fläche des Verschlusses und insbesondere die Fläche des Verschlusses, die aus quellbarem und/oder die Flüssigkeit transportierenden Material besteht, wird bevorzugterweise durch auswechselbare Elemente bestimmt, die an der Innenseite des Verschlusses angeordnet sind. Diese auswechselbaren Elemente haben eine oder mehrere unterschiedlich große Öffnungen, so dass je nach auswechselbarem Element eine bestimmte Fläche des Verschlusses und insbesondere Fläche des Verschlusses, die aus quellbarem und/oder die Flüssigkeit transportierenden Material besteht, vorgegeben wird, die in direktem Kontakt mit der Flüssigkeit steht. Dadurch kann die Verdunstungsrate reguliert werden.

Alternativ wird die Verdunstungsrate durch unterschiedliche Verschlüsse bestimmt, die je nach gewünschter Verdunstungsrate unterschiedlich große Verdunstungsoberflächen für die Verdunstungseinrichtung bereitstellen. Damit ist es möglich, die Verdunstungsrate der Vorrichtung an unterschiedliche Raumvolumina anzupassen.

Bei einer weiteren Ausgestaltung, bei der ein langgestrecktes Element insbesondere ein Stab oder Docht oder Tampon oder Kombinationen aus diesen zum Transportieren der Flüssigkeit zu der Dichteinrichtung und/oder der Verdunstungseinrichtung an dem Verschluss verwendet wird, kann bevorzugterweise die Verdunstungsrate dadurch bestimmt werden, dass der Stab oder der Docht oder der Tampon oder eine Kombination aus solchen Materialien eine entsprechende Form aufweist. Beispielsweise werden unterschiedliche Verdunstungsraten durch unterschiedliche Durchmesser des Stabes oder des Dochtes oder des Tampons oder einer Kombination aus solchen Materialien vorgegeben. Alternativ können unterschiedliche Materialen für das langgestreckte Element verwendet werden, wie zum Beispiel ein Docht für eine schnelle Verdunstungsrate oder ein Stab aus Holz für geringere Verdunstungsraten. Ebenfalls sind Stäbe aus unterschiedlichen Materialen wie zum Beispiel unterschiedlichen Holzarten möglich, die die Flüssigkeit unterschiedlich schnell transportieren.

In einer anderen Ausgestaltung ist es vorgesehen, dass der Behälter von einem Hüllkörper umgeben ist. Dieser Hüllkörper weist einen Hohlraum auf, in den der Behälter zumindest teilweise formschlüssig eingesetzt werden kann. Dies hat den Vorteil, dass der Behälter vor Zerbrechen geschützt ist. Weiterhin kann der Hüllkörper so gestaltet werden, dass ein Öffnen leichter möglich ist, da der Behälter fest, insbesondere drehfest, in dem Hüllkörper gehalten wird.

In einer weiteren Ausgestaltung umfasst die Vorrichtung weiterhin eine Flüssigkeit, insbesondere einen Duftstoff zusammen mit Alkohol. Bei der Flüssigkeit kann es sich insbesondere um Flüssigkeitsgemische aus zumindest zwei Komponenten handeln, beispielsweise ätherisches Öl in Alkohol. Ebenso können ätherische Öle separat oder weitere flüssige Duftstoffe verwendet werden. Die Vorrichtung zum Verdunsten von Flüssigkeit kann damit insbesondere zum Abgeben von Gerüchen oder Duft verwendet werden.

Die Duft abgebende Flüssigkeit ist also insbesondere ein ätherisches Öl bzw. eine Komposition verschiedener ätherischer Öle. Diese verströmen unterschiedliche Aromen und verdampfen im Wesentlichen rückstandslos. Zur Steuerung der Dauer der Duftabgabe bzw. der Transporteigenschaften werden die ätherischen Öle mit Alkohol gemischt. Als besonders geeignet hat sich dabei ein Alkohol-Anteil von 25% bis 40% (Gewichtsprozent) erwiesen. Für bestimmte ätherischen Öle bzw. für bestimmte Verwendungszwecke der ätherischen Öle kann der Alkohol-Anteil auf ungefähr 60% oder sogar ungefähr 80% gesteigert werden. Die Menge des in der Mischung enthaltenen Alkohols steuert die Oberflächenspannung der Flüssigkeit und wirkt sich damit auf die Transporteigenschaften der verwendeten Hölzer aus. Auf dieser Basis kann somit die Dauer der Duftabgabe beeinflusst werden.

Gemäß einem weiteren Aspekt umfasst die Erfindung einen Verschluss für eine Vorrichtung zum Verdunsten von Flüssigkeit.

Die oben genannten Ausgestaltungen oder/und Ausführungsformen können miteinander kombiniert werden oder teilweise kombiniert oder alternativ verwendet werden. Die Erfindung umfasst auch alle sinnvollen und insbesondere alle erwähnten Kombinationen von unabhängigen und/oder abhängigen Ansprüchen.

Weitere Einzelheiten und Merkmale ergeben sich aus der nachfolgenden Beschreibung bevorzugter Ausführungsbeispiele auch in Verbindung mit den Unteransprüchen. Hierbei können die jeweiligen Merkmale für sich alleine oder zu mehreren in Kombination miteinander verwirklicht sein. Die Möglichkeiten, die Aufgabe zu lösen, sind nicht auf das Ausführungsbeispiel beschränkt. So umfassen beispielsweise Bereichsangaben stets alle - auch nicht genannten - Zwischenwerte und alle denkbaren Teilintervalle.

Die Ausführungsbeispiele sind teilweise in den Figuren schematisch dargestellt. Gleiche Bezugsziffern in den einzelnen Figuren bezeichnen dabei gleiche oder funktionsgleiche bzw. hinsichtlich ihrer Funktionen einander entsprechende Elemente. Im Einzelnen zeigen:
- Fig. 1A: eine Ansicht des hölzernen Verschlusses von unten (schematisch) gemäß einer bevorzugten Ausführungsform der vorliegenden Erfindung;
- Fig. 1B: eine seitliche Schnittansicht des Verschlusses gemäß Fig. 1A;
- Fig. 1C: eine um 90° gedrehte seitliche Schnittansicht des Verschlusses gemäß Fig. 1A;
- Fig. 2A: eine Seitenansicht des hölzernen Verschlusses (Außenansicht, schematisch) gemäß einer bevorzugten Ausführungsform der vorliegenden Erfindung;
- Fig. 2B: eine ggü. Fig. 2A um 90° gedrehte Seitenansicht des Verschlusses (Außenansicht, schematisch);
- Fig. 3: eine schematische seitliche Schnittansicht des Verschlusses mit schematischer Darstellung der Zustände vor und nach dem Quellvorgang gemäß einer bevorzugten Ausführungsform der vorliegenden Erfindung;
- Fig. 4: eine schematische seitliche Schnittansicht des Flakons mit aufgesetztem Verschluss gemäß einer bevorzugten Ausführungsform der vorliegenden Erfindung.

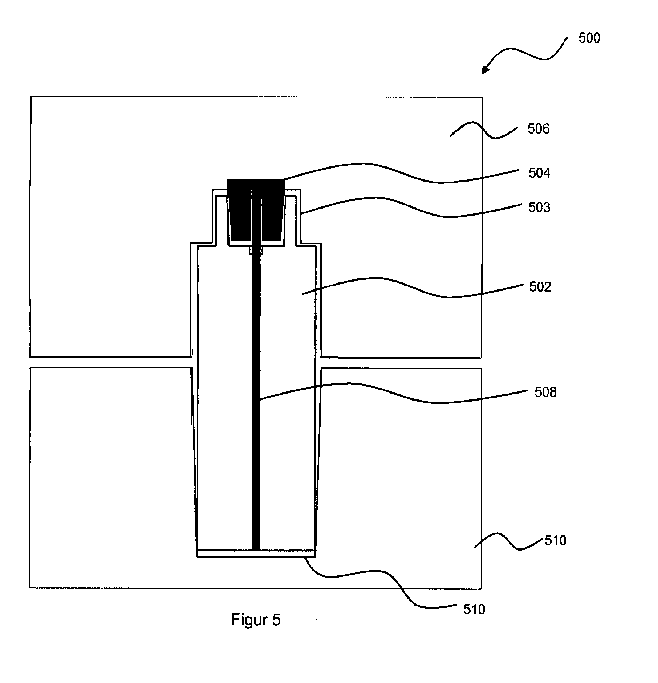

Ein weiteres Ausführungsbeispiel ist in den Fig. 5 bis 7 gezeigt.

Es zeigen:
- Fig. 5: einen Querschnitt durch die Vorrichtung zur Verdunstung gemäß einer bevorzugten Ausführungsform der vorliegenden Erfindung;

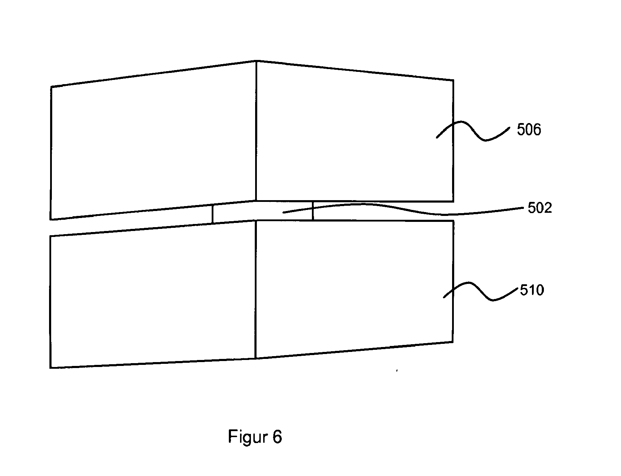

- Fig. 6: eine perspektivische Ansicht der Vorrichtung zur Verdunstung gemäß einer bevorzugten Ausführungsform der vorliegenden Erfindung, bei der der Deckel aufgesetzt ist;

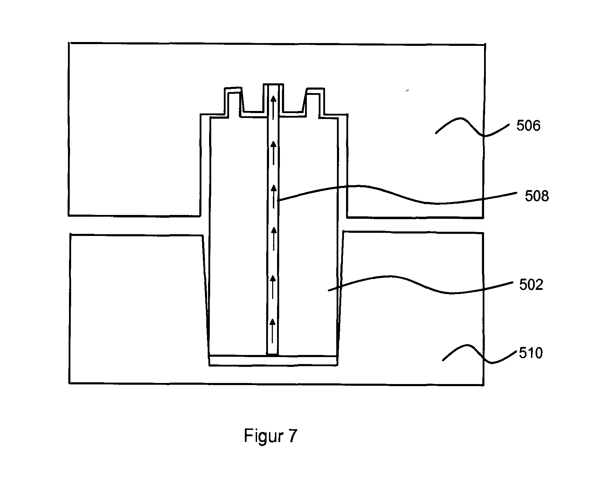

- Fig. 7: einen Querschnitt der Vorrichtung gemäß einer bevorzugten Ausführungsform der vorliegenden Erfindung, wobei der Weg der Flüssigkeit angedeutet ist.

Weitere Ausführungsbeispiele sind in den folgenden Figuren dargestellt:

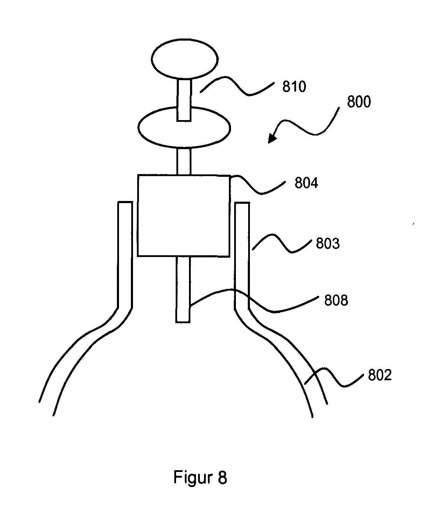

- Fig. 8: zeigt eine Verdunstungsvorrichtung gemäß einer bevorzugten Ausführungsform der vorliegenden Erfindung, bei der ein weiteres Element in Form einer Blume am Verschluss angebracht ist;

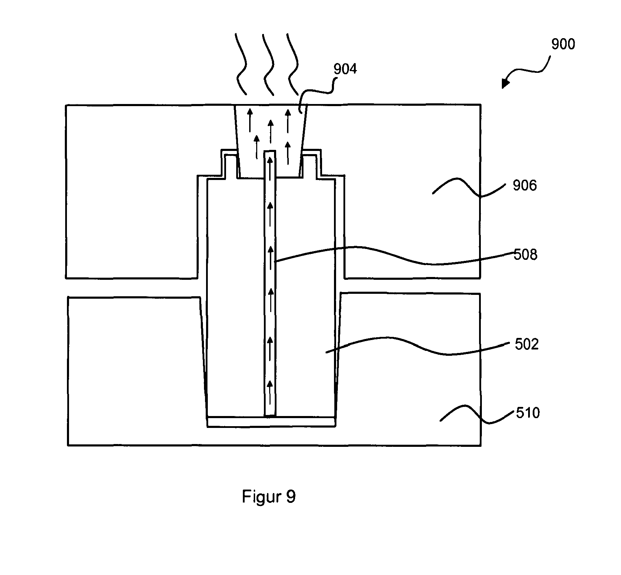

- Fig. 9: zeigt eine alternative Ausführungsform zur Fig. 5, bei der der Flüssigkeitstransport nur im ersten Teil des Verschlusses erfolgt;

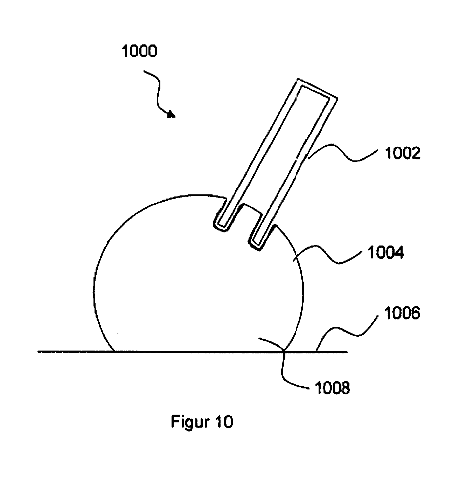

- Fig. 10: zeigt eine Verdunstungsvorrichtung gemäß einer bevorzugten Ausführungsform der vorliegenden Erfindung, die auf den Verschluss gestellt ist; und

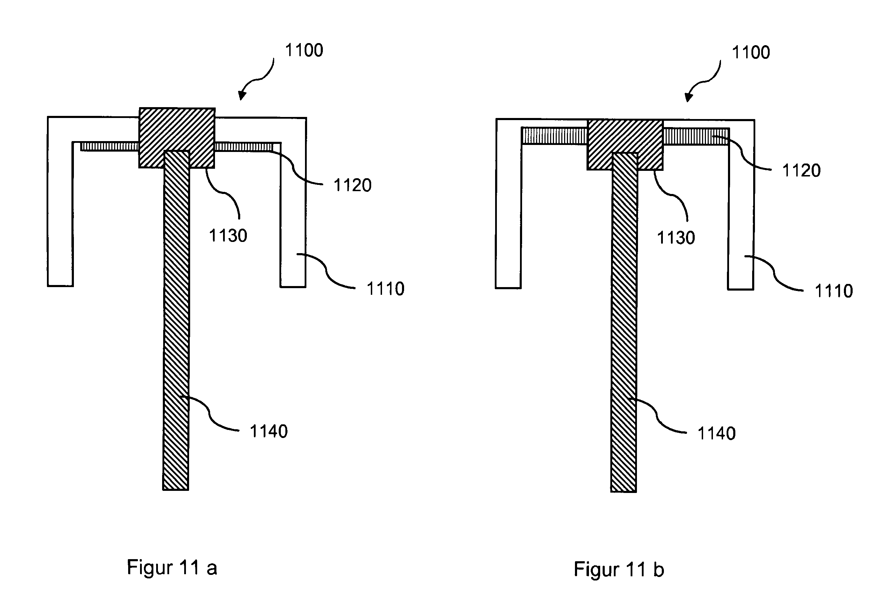

- Fig. 11A bis 11F: zeigen schematische Darstellungen verschiedener Ausgestaltungen des Verschlusses der Verdunstungsvorrichtung gemäß einer weiteren bevorzugten Ausführungsform der vorliegenden Erfindung.

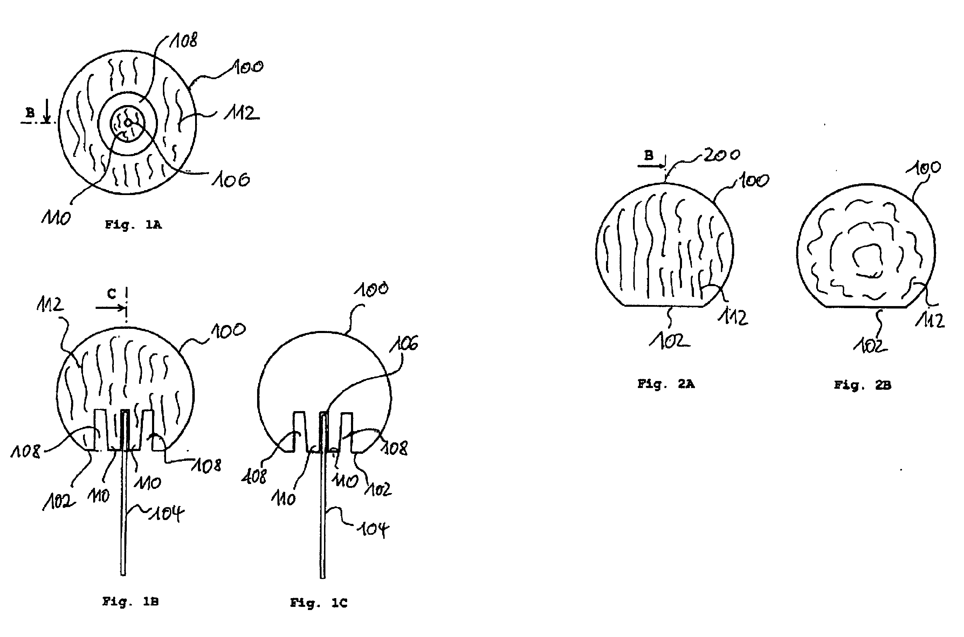

Fig. 1A bis 1C zeigen den hölzernen Verschluss der Duft abgebenden Vorrichtung in verschiedenen Ansichten (schematisch), die jeweils um 90° gegeneinander verkippt bzw. abgerollt sind. Der Verschluss 100 besteht aus Erlenholz und weist (in diesen Figuren) eine kugelähnliche Form auf. An der Unterseite mit Richtung zum Flakon weist er eine geringfiigig abgeplattete Form 102 auf.

Zur Aufnahme des hölzernen Stabes 104 ist eine zentrale Bohrung 106 vorhanden, in die der Stab 104 eingesetzt wird. Weiterhin weist der Verschluss 100 eine ringförmige Fräsung 108 auf, deren Radius sich in Richtung zum Inneren des Verschlusses hin verringert (konischer Verlauf). Zwischen der zentralen Bohrung 106 zur Aufnahme des Stabes 104 und der ringförmigen Ausfräsung 108 befindet sich ein verbliebener hölzerner Ring 110. Die Länge des hölzernen Stabes 104 ist so gewählt, dass der Stab 104 nach dem Aufsetzen des Verschlusses 100 auf den Flakon bis fast zum Boden des Flakons reicht, um die Flüssigkeit möglichst vollständig zum Verschluss transportieren zu können.

Die Anordnung der zentralen Bohrung 106 ist so gewählt, dass sie in der Ebene der Maserung 112 und senkrecht zur Richtung der (ehemaligen) Baumspitze verläuft.

Die Figuren 2A und 2B zeigen verschiedene Ansichten der Oberflächen des Verschlusses 100, wobei diese beiden Ansichten des Verschluss 100 zueinander jeweils um 90° um die vertikale Achse 200 gedreht sind. Diese beiden Figuren zeigen insbesondere den Verlauf der natürlichen Maserung 112 des Holzes.

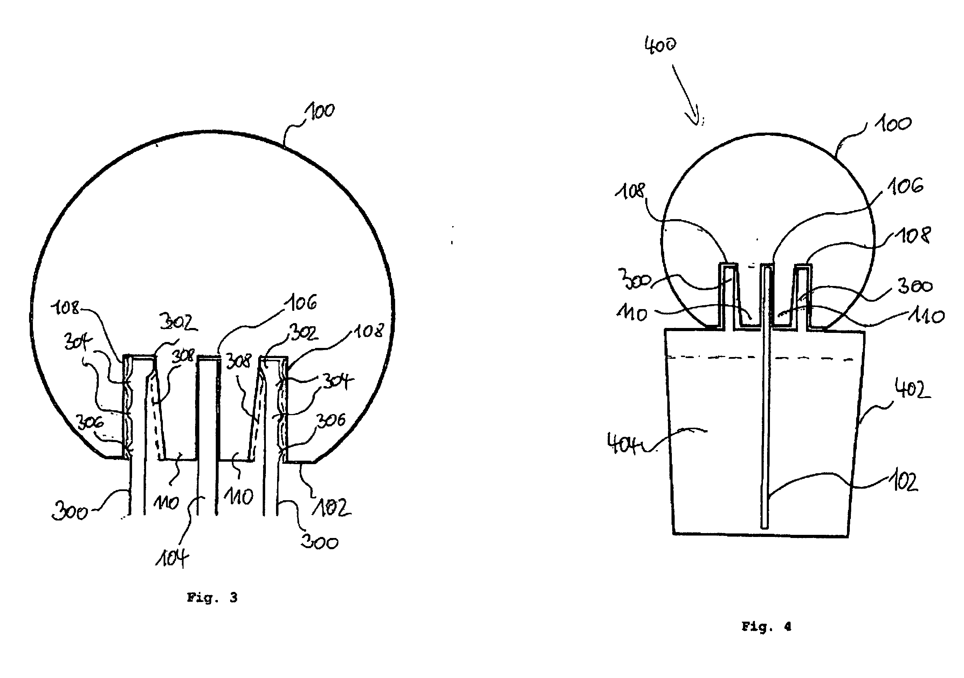

Die schematische Detailansicht des Verschlusses 100 in Fig. 3 zeigt den Hals 300 des Flakons mit dem aufgesetzten Verschluss 100. In der zentralen Bohrung 106 befindet sich der hölzerne Stab 104. Der Hals 300 des Flakons weist an seinem Ende am Innenradius eine Wulst 302 auf. Die äußere Oberfläche des Halses 300 ist beispielsweise mit einem Gewinde 304 und einer ringförmigen Wulst 306 versehen. Der aufgesetzte hölzerne Verschluss 100 ist am oberen Ende der konisch zulaufenden Fräsung 108 an der Wulst 302 am Innenrand des Halses 300 des Flakons verklemmt und somit kraftschlüssig mit diesem verbunden. Das Quellen des hölzernen Verschlusses 100 bewirkt das Entstehen einer formschlüssigen Verbindung mit dem Hals 300 des Flakons, dargestellt durch den gestrichelten Verlauf 308 der Oberfläche des Verschlusses 100 im Bereich der Fräsung 108.

Die Darstellung in Fig. 4 zeigt schematisch die Duft abgebende Vorrichtung 400 mit dem Flakon 402 und dem aufgesetzten hölzernen Verschluss 100. In die zentrale Bohrung 106 ist der Stab 104 eingesetzt. Dieser reicht in die Duft abgebende Flüssigkeit 404 hinein und transportiert sie in den Verschluss 100. Der Flüssigkeitspegel 406 ist angedeutet.

In Fig. 5 ist eine Vorrichtung zur Verdunstung 500 im Querschnitt dargestellt. Die Vorrichtung weist einen Behälter 502 auf, der einen Hals 503 als Behälteröffnung aufweist. In den Behälter ist eine flüssige Substanz eingefüllt, die bei Verdunsten einen Geruch, beispielsweise einen Duft, abgeben kann. Der Verschluss umfasst einen ersten Teil 504 des Verschlusses und einen zweiten Teil 506 des Verschlusses. Der erste Teil 504 des Verschlusses ist aus quellbarem Material gefertigt. Dadurch sitzt es, sobald es mit Flüssigkeit benetzt wird, dicht im Behälterhals 503 und gewährleistet so eine Abdichtung des Behälters. Alternativ oder zusätzlich ist der zweite Teil des Verschlusses 506 aus quellbarem Material gefertigt. Im Falle des Kontaktes mit Flüssigkeit dehnt es sich aus und sitzt so kraftschlüssig an der Außenseite des Behälters 502 und verhindert damit ein Austreten von Flüssigkeit.

Die Vorrichtung 500 weist weiterhin einen Stab 508 auf, der in den ersten Teil des Verschlusses 504 eingesetzt ist. Der Stab 508 reicht im Wesentlichen bis zum Boden des Behälters 502, sodass über Kapillarwirkung Flüssigkeit aus dem Behälter bis zu dessen Entleerung gezogen werden kann. Weiterhin weist die Vorrichtung 500 einen Hüllkörper 510 auf, in dem ein Hohlraum 512 ausgebildet ist. Der Hohlraum 512 ist so bemessen, dass der Behälter zumindest teilweise formschlüssig am Hüllkörper 510 anliegt.

Fig. 6 zeigt diese Vorrichtung 500 bei aufgesetztem Verschluss in perspektivischer Darstellung. Der Abstand zwischen dem zweiten Teil des Verschlusses 506 und dem Hüllkörper 510 kann insbesondere so gewählt werden, dass ein optimaler Schutz des Behälters 502 vor Zerbrechen oder auch vor zu starker Erwärmung, z.B. durch Sonneneinstrahlung, gewährleistet ist.

Die Lage des Schlitzes zwischen Hüllkörper 510 und dem zweiten Teil des Verschlusses 506, also die Abmessungen von Hüllkörper 510 und vom zweiten Teil des Verschlusses 506, können so gewählt werden, dass durch den Schlitz der untere Teil des Behälters 502 von außen sichtbar ist und damit ein niedriger Füllstand des Behälters 502 festgestellt werden kann.

In Fig. 7 ist durch Pfeile entlang des Stabs 508 der Weg der Flüssigkeit angedeutet. Die Flüssigkeit wird also entlang des Stabs 508 nach oben transportiert und tritt durch den ersten Teil des Verschlusses 504 in den zweiten Teil des Verschlusses 506 über und wird darin zu dessen Oberfläche transportiert, wo sie verdunstet. Durch die Verdunstung dort wird weitere Flüssigkeit aus dem Behälter 502 gesogen.

Alternativ erfolgt in dem Ausführungsbeispiel gemäß Fig. 9 der Flüssigkeitstransport nur in dem ersten Teil des Verschlusses 904. Der zweite Teil des Verschlusses weist also keine wesentlichen Transporteigenschaften für Flüssigkeit auf. Damit lässt sich die Verdunstung auf bestimmte Oberflächenbereiche einschränken.

In Fig. 8 ist eine weitere Vorrichtung zur Duftabgabe 800 dargestellt. Der Behälter 802 weist einen Behälterhals 803 auf. Der Behälterhals 803 wird durch einen Verschluss 804 aus quellbarem Material verschlossen. Um die Verdunstungsrate auf ein gewünschtes Maß einzustellen, wird Flüssigkeit aus dem Behälter 802 über einen Stab 808 in den Verschluss 804 und in ein als Blume ausgestaltetes weiteres Verdunstungselement 810 transportiert. Durch die Wahl der Größe der Oberfläche des weiteren Verdunstungselements 810 kann eine bestimmte Verdunstungsrate erzielt werden.

Alternativ zu der in Fig. 8 dargestellten Version der Ausführungsform weist bei einer weiteren Ausgestaltung der Ausführungsform das Verdunstungselement 810 eine oder mehrere Bohrungen auf, die einen oder mehrere Stäbe aufnehmen. Diese eine oder mehreren Stäbe bestehen bevorzugterweise aus flüssigkeitstransportierendem Material und nehmen Flüssigkeit von dem Verdunstungselements 810 auf. Bevorzugterweise sind an den Stäben weitere Elemente angeordnet, die die Verdunstung der von dem einen oder mehreren Stäbe transportierten Flüssigkeit übernehmen oder unterstützen. Die einen oder mehreren Stäbe sind wahlweise so ausgebildet, dass sie über ihre Oberfläche die transportierte Flüssigkeit verdunsten, oder sie sind beschichtet, so dass die transportierte Flüssigkeit über ihre Oberfläche nicht verdunsten. Zur Dekorationszwecken können an dem einen oder mehreren Stäben z.B. eine oder mehrere Stoffblumen befestigt werden. Bevorzugterweise sind die Stoffblumen so an den jeweiligen Stäben befestigt, dass sie keine Flüssigkeit aufnehmen. Dies schützt den Stoff der Stoffblumen davor, von der Flüssigkeit benetzt zu werden. Alternativ werden die Stoffblumen mit Textilien, die nicht durch die Flüssigkeit beschädigt werden, derart an den jeweiligen Stäben befestigt, dass sie ebenfalls die Flüssigkeit aufnehmen und verdunsten. Statt Stoffblumen können auch andere Textilelement eingesetzt werden.

In Fig. 10 ist eine weitere Vorrichtung 1000 zur Verdunstung von Flüssigkeit gezeigt. Der Behälter 1002 ist mit dem Verschluss 1004 umgedreht aufgestellt, indem der Verschluss 1004 eine ebene Fläche 1008 aufweist, die auf einer Unterlage 1006 derart aufliegt, dass die Vorrichtung in dieser Stellung verharrt. Damit die Unterlage 1006 nicht beschädigt wird, ist die ebene Fläche 1008 mit einer Schicht aus flüssigkeitsundurchlässigem Material versehen, beispielsweise einer Lackschicht oder der Verschluss 1004 weist hier einen Bereich aus einem nicht flüssigkeitsdurchlässigen Material auf. Dazu kann der Verschluss 1004 wie in dem Ausführungsbeispiel in Fig. 9 aus flüssigkeitstransporierenden und nicht flüssigkeitstransporierenden Materialien gebildet sein. Alternativ oder zusätzlich wird die ebene Fläche 1008 auf einen Untersetzer gestellt.

Figuren 11 A bis 11 F zeigen schematische Darstellungen verschiedener Ausgestaltungen des Verschlusses der Verdunstungsvorrichtung gemäß einer weiteren bevorzugten Ausführungsform der vorliegenden Erfindung.

Der Verschluss 1100, der bevorzugterweise als Kappe ausgebildet ist, weist dabei die Verschlusskappe 1110, die Dichtungseinrichtung 1120, die Verdunstungseinrichtung 1130 und das langgestreckte Element 1140 auf.

Entsprechend den verschiedenen Ausgestaltungen dieser bevorzugten Ausführungsformen weist die Verschlusskappe 1110 ein innenseitiges Schraubgewinde auf, das in den Figuren 11 A bis 11 F der Übersichtlichkeit wegen nicht dargestellt ist. Alternativ kann die Kappe 1110 innenliegend Vorsprünge aufweisen, die an der Außenseite der Behälteröffnung bevorzugterweise kraftschlüssig anliegen, um einen sicheren Sitz des Verschlusses 1100 auf der Behälteröffnung zu gewährleisten. Bei Aufquellen der Dichteinrichtung 1120 wird der Kraftschluss durch die Volumenänderung der Dichteinrichtung 1120 verstärkt und die Vorrichtung zum Verdunsten von Flüssigkeiten gegen unbeabsichtigtes Öffnen gesichert.

An der der Behälteröffnung zugewandten Innenseite des Verschlusses 1100 ist bevorzugterweise die Dichteinrichtung 1120 angeordnet. Bevorzugterweise besteht sie vollständig aus quellbarem Material und ist so angeordnet, dass sie beim Aufsetzen des Verschlusses auf die Vorrichtung mit dem Rand der Behälteröffnung im Wesendlichen vollständig in Kontakt ist.

Außerdem weist der Verschluss 1100 die Verdunstungseinrichtung 1130 auf, die bevorzugterweise mittig in der Verschlusskappe angeordnet ist. Bevorzugterweise ist die Verdunstungseinrichtung 1130 in eine mittige Öffnung der Verschlusskappe 1110 eingebracht und in Kontakt mit der Dichteinrichtung 1120. Die Verdunstungseinrichtung 1130 besteht dabei bevorzugterweise aus flüssigkeitstransportierendem Material, wie z. B. poröser Kunststoff, Zellstoff, Holz, etc., und schließt dabei eben mit der Außenseite der Verschlusskappe 1110 ab. Alternativ steht die Verdunstungseinrichtung 1130, wie in Figur 11 A gezeigt, über die Außenseite der Verschlusskappe 1110 hinaus. Durch den Kontakt des flüssigkeitstransportierenden Materials der Verdunstungseinrichtung 1130 mit der Dichteinrichtung 1120 wird die Dichteinrichtung 1120 mit Flüssigkeit benetzt und kann so aufquellen. Eine derartige Anordnung stellt sicher, dass während des Verdunstungsbetriebes der erfindungsgemäßen Vorrichtung die Behälteröffnung mittels Dichteinrichtung sicher gegen unbefugtes Öffnen verschlossen ist. Die Dichteinrichtung 1120 kann dabei eine ringförmige Scheibe sein, die bevorzugterweise formschlüssig um die Verdunstungseinrichtung 1130 angeordnet ist.

Darüber hinaus verfügt der erfindungsgemäße Verschluss 1100 über ein langgestrecktes Element 1140, das bevorzugterweise eine längliche Zylinderform aufweist und mit der Verdunstungseinrichtung gekoppelt ist. Bevorzugterweise ist die Länge des langgestreckten Elements 1140 so bemessen, dass bei Befestigung des Verschlusses 1100 auf der Behälteröffnung das langgestreckte Element 1140 bis auf den Boden des Behälters reicht. Das langgestreckte Element 1140 besteht bevorzugterweise aus flüssigkeitstransportierenden Material und wird bevorzugterweise durch einen Holzstab, einen Docht oder einen stabförmigen Tampon gebildet. Alternativ besteht das langgestreckte Element 1140 aus quellbarem Material, das darüber hinaus auch geeignet ist, Flüssigkeit zu transportieren. Durch die Kopplung des langgestreckten Elements 1140 mit der Verdunstungseinrichtung 1130 wird somit die Verdunstungseinrichtung 1130 und die Dichteinrichtung 1120 mit Flüssigkeit aus dem Behälterinneren versorgt.

Figur 11 B zeigt eine weitere Ausführungsform des Verschlusses 1100, die sich dadurch von Figur 11 A unterscheidet, dass die Dichteinrichtung in der Verschlusskappe 1110 angeordnet ist.

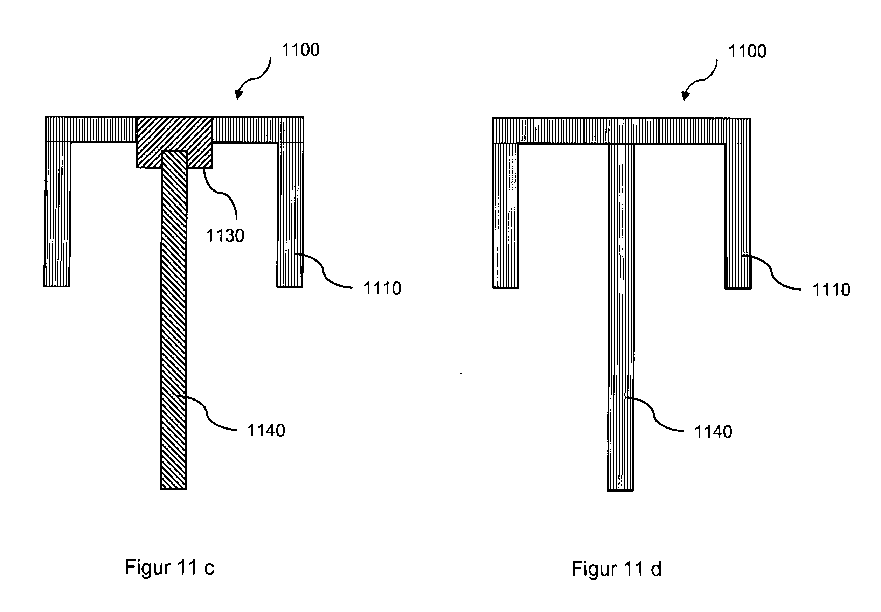

Figur 11 C zeigt eine weitere Version des in Figur 11 A dargestellten Verschlusses 1100. Bei der in Figur 11 C gezeigten Version des Verschlusses 1100 ist die Verschlusskappe 1110 aus quellbarem Material, wie z. B. Holz, gefertigt. Damit bilden zumindest Teile der Verschlusskappe 1110 die Dichteinrichtung 1120. Durch das Quellen einer oder mehrerer der Behälteröffnung zugewandten Innenseiten der Verschlusskappe 1110 wird ein flüssigkeitsdichter Verschluss der Behälteröffnung gewährleistet und der Behälter gegen ungefugtes Öffnen gesichert.

Figur 11 D zeigt eine weitere Ausgestaltungsform des in Figur 11 A gezeigten Verschlusses 1100. In der in Figur 11D gezeigten Version des Verschlusses 1100 ist die Verschlusskappe 1110 einstückig mit der Dichteinrichtung 1120, der Verdunstungseinrichtung 1130 und dem langgestreckten Element 1140 ausgebildet. Bevorzugterweise besteht dieser einstückig ausgebildete Verschluss aus einer oder mehreren Holzarten bzw. aus einem oder mehreren quellbaren Kunststoffen. Alternativ ist das langgestreckte Element 1140 in die einstückig ausgebildete Verschlusskappe 1110 einsteckbar. Bei dieser Version des Verschlusses 1100 dient zumindest ein Teil der Innenseite der Verschlusskappe 1110 als Dichteinrichtung 1120 und ein Teil der Außenseite der Verschlusskappe 1110 als Verdunstungseinrichtung 1130.

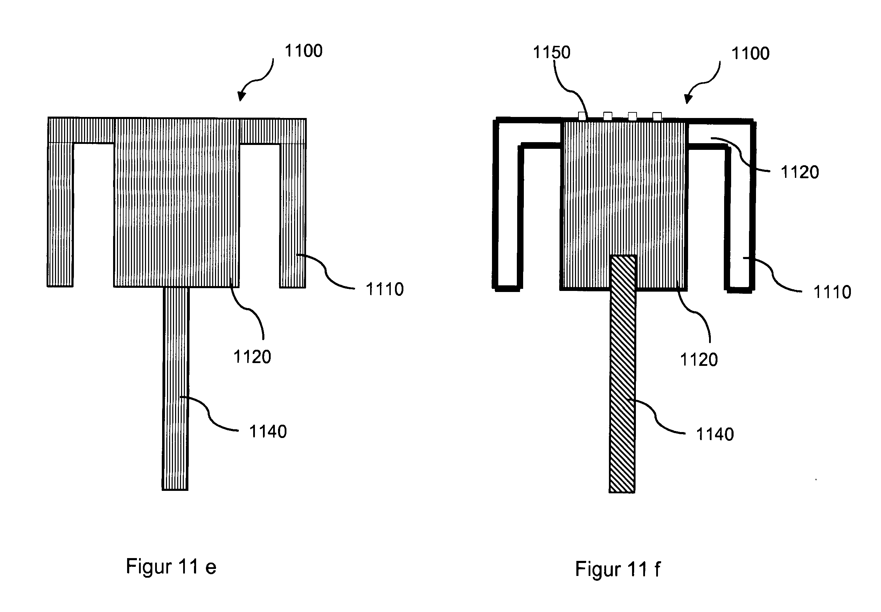

Wie in Figuren 11 E und 11 F gezeigt, ist in einer weiteren Version des in Figur 11 A gezeigten Verschlusses 1100 die Dichteinrichtung 1120 zylinderförmig ausgestaltet, wobei sich die Dichteinrichtung 1120 in Richtung der Behälteröffnung erstreckt. Der Durchmesser der im Wesentlichen zylinderförmigen Dichteinrichtung 1120 ist dabei so gewählt, dass die Dichteinrichtung 1120 bereits in nichtgequollenem Zustand die Behälteröffnung flüssigkeitsdicht abschließt. Durch Aufquellen der Dichteinrichtung 1120 wird die Behälteröffnung gegen unbeabsichtigtes Öffnen gesichert.

Alternativ kann anstelle einer zylinderförmigen Dichteinrichtung jede andere, an die Behälterform angepasste Dichteinrichtung verwendet werden.

Wie darüber hinaus in Figur 11 E gezeigt, bestehen sowohl die Verschlusskappe 1110 als auch das langgestreckte Element 1140 aus quellbarem Material. Bevorzugterweise wird hierfür eine oder mehrere verschiedene Holzarten verwendet. Die Verdunstungseinrichtung wird hierbei zumindest zum Teil durch die Außenoberfläche der Verschlusskappe 1110 gebildet.

Figur 11 F zeigt eine weitere Ausgestaltungsform der in Figur 11 E gezeigten Version des Verschlusses 1100. Der Verschluss 1100 ist dabei aus unterschiedlichen Materialien zusammengesetzt. Bevorzugterweise besteht dabei die Verschlusskappe 1110 im Wesentlichen aus nicht quellbarem und nicht flüssigkeitstransportierenden Material. Die Dichteinrichtung 1120 und die Verdunstungseinrichtung 1130 werden durch einen quellbaren Körper gebildet, der im Zentrum der Verschlusskappe angeordnet ist. Bevorzugterweise weist dieser Körper eine zylindrische Form auf und ist mit einem Material überzogen, dass nicht quellbar und nicht flüssigkeitstransportierend ist. Damit die Verdunstungseinrichtung 1130 die Flüssigkeit entsprechend verdunsten kann, weist sie in dem Überzug aus nicht quellbarem und nicht flüssigkeitstransportierenden Material an der Außenseite des Verschlusses 1100 Öffnungen 1150 auf, die ein Austreten der verdunsteten Flüssigkeit erlauben.

Die Flüssigkeit wird mittels des flüssigkeitstransportierenden, langgestreckten Elements 1140 zu der Dicht- und Verdunstungseinrichtung 1120/1130 befördert. Typischerweise weist die Dicht- und Verdunstungseinrichtung 1120/1130 eine Bohrung auf, die das langgestreckte Element 1140 aufnimmt. Die Dicht- und Verdunstungseinrichtung 1120/1130 besteht dabei bevorzugterweise aus Holz oder Zellstoff oder Kork oder quellbarem Kunststoff oder einer Kombination aus diesen Materialien.

Mittels der vorliegenden Erfindung ist es möglich durch einen Verschluss mit wenigen Einzelelementen eine Vorrichtung zur Verdunstung einer Flüssigkeit anzugeben, die während des Verdunstungsbetriebes einerseits den Behälter der erfindungsgemäßen Vorrichtung flüssigkeitsdicht verschließt und andererseits ein unbefugtes Öffnen des Behälters im Wesentlichen verhindert, zumindest aber erschwert. Je nach Wahl des quellbaren Materials kann dann nach Beendigung des Verdunstungsbetriebes, z.B. wenn die zu verdunstende Flüssigkeit aufgebraucht ist, der Behälter der erfindungsgemäßen Vorrichtung wieder einfach geöffnet werden, um z. B. zu verdunstende Flüssigkeit nachzufüllen.

Die Erfindung gibt somit eine Vorrichtung zum Verdunsten von Flüssigkeit mit einem Verschluss an, der zumindest, solange die Vorrichtung Flüssigkeit hält, insbesondere nicht ungewollt durch Kinder geöffnet werden kann. Die Vorrichtung umfasst einen Behälter zum Aufbewahren von Flüssigkeit und einen Verschluss der auf eine Behälteröffnung gesetzt werden kann. Zumindest Teile des Verschlusses bestehen aus einem Material, das durch Kontakt mit Flüssigkeit aufquillt. Damit wird der Kontakt zwischen Verschluss und Behälteröffnung so verändert, dass ein Öffnen des Verschlusses erschwert wird. Weiterhin umfasst die Vorrichtung eine Verdunstungseinrichtung, die insbesondere in den Verschluss integriert ist oder/und das quellbare Material umfasst.

Eine Ausführungsform der Erfindung umfasst eine Vorrichtung zum Verdunsten von Flüssigkeit mit einem Behälter zum Aufnehmen einer Flüssigkeit, der zumindest eine Behälteröffnung aufweist, und einem Verschluss mit einer Dichteinrichtung zum Verschließen der Behälteröffnung und einer Verdunstungseinrichtung, wobei die Dichteinrichtung zumindest teilweise aus einem Material besteht, das durch Kontakt mit der Flüssigkeit quellbar ist.

Bevorzugterweise ist das quellbare Material derart beschaffen, dass die Flüssigkeit durch das quellbare Material transportierbar ist.

Bei einer bevorzugten Weiterbildung ist die Dichteinrichtung durch zumindest einen Vorsprung an der zur Behälteröffnung gerichteten Seite des Verschlusses gebildet, der so ausgestaltet ist, dass der Vorsprung an der Behälteröffnung auf der Außenseite oder/und Innenseite oder/und Oberseite des Behälters kraftschlüssig anliegt.

Bei einer bevorzugten Weiterbildung sind die Dichteinrichtung und die Verdunstungseinrichtung des Verschlusses derart gekoppelt, dass sie beide direkt oder indirekt mit der Flüssigkeit benetzbar sind.

Bei einer bevorzugten Weiterbildung weist der zumindest eine Vorsprung zumindest eine Riffelung oder/und die Behälteröffnung eine zumindest teilweise korrespondierende Riffelung auf.

Bei einer bevorzugten Weiterbildung verjüngt sich der Innendurchmesser der Behälteröffnung zur Außenseite des Behälters hin.

Bei einer bevorzugten Weiterbildung ist die Dichteinrichtung einstückig mit dem Verschluss gebildet ist und der Verschluss und die Dichteinrichtung bestehen im Wesentlichen aus quellbarem Material.

Bei einer bevorzugten Weiterbildung umfasst die Vorrichtung weiter ein an dem Verschluss befestigtes, langgestrecktes Element und die Flüssigkeit ist über das Element in das quellbare Material des Verschlusses transportierbar.

Bei einer bevorzugten Weiterbildung ist das Material, das der Verschluss zumindest teilweise umfasst, Holz oder quellbarer Kunststoff.

Bei einer bevorzugten Weiterbildung ist das langgestreckte Element in eine Bohrung im Verschluss einsetzbar und die Bohrung liegt in der Ebene der Maserung des Holzes des Verschlusses.

Bevorzugterweise ist zumindest ein weiteres Element am Verschluss befestigbar.

Bei einer bevorzugten Weiterbildung weist der Verschluss zumindest eine ebene äußere Fläche auf.

Bei einer bevorzugten Weiterbildung ist der Behälter von einem Hüllkörper umgeben, in dem die Form des Behälters ausgenommen ist, sodass der Behälter zumindest teilweise formschlüssig in den Hüllkörper setzbar ist.

Bei einer bevorzugten Weiterbildung umfasst die Vorrichtung weiter eine Flüssigkeit, enthaltend einen Duftstoff und 25% bis 40% Gewichtsprozent Alkohohl.

Eine weitere Ausführungsform der Erfindung ist ein Verschluss für eine Vorrichtung zum Verdunsten von Flüssigkeit mit einer Dichteinrichtung zum Verschließen einer Behälteröffnung eines zumindest teilweise mit Flüssigkeit befüllten Behälters, wobei die Quelldichtung zumindest teilweise aus einem Material besteht, das durch Kontakt mit der Flüssigkeit quellbar ist, und der Verschluss weiterhin eine Verdunstungseinrichtung umfasst.

## Patentansprüche

1. Vorrichtung zum Verdunsten von Flüssigkeit, wobei die Vorrichtung umfasst:
einen Behälter (402, 502) zum Aufnehmen einer Flüssigkeit (404), der zumindest eine Behälteröffnung (300, 503) aufweist,
einem Verschluss (100, 504, 506, 804, 904, 906, 1100) mit einer Dichteinrichtung zum Verschließen der Behälteröffnung (300, 503, 803) und mit einer Verdunstungseinrichtung zum Verdunsten der Flüssigkeit, wobei die Dichteinrichtung zumindest teilweise aus einem Material besteht, das durch Kontakt mit der Flüssigkeit (404) quellbar ist, wobei durch das Quellen zumindest abschnittsweise eine kraftschlüssige Verbindung zwischen Behälteröffnung (300, 503) und Verschluss (100, 504, 506, 804, 904, 906, 1100) hergestellt oder verstärkt wird.

2. Vorrichtung nach Anspruch 1, wobei das quellbare Material derart beschaffen ist, dass die Flüssigkeit (404) durch das quellbare Material transportierbar ist.

3. Vorrichtung nach Anspruch 1 oder 2, bei der die Dichteinrichtung durch zumindest einen Vorsprung an der zur Behälteröffnung (300, 503) gerichteten Seite des Verschlusses (100, 504, 506, 804, 904, 906) gebildet ist, der so ausgestaltet ist, dass der Vorsprung an der Behälteröffnung (300, 503) auf der Außenseite oder/und Innenseite oder/und Oberseite des Behälters (402, 502) kraftschlüssig anliegt.

4. Vorrichtung nach einem der vorhergehenden Ansprüche, bei der die Dichteinrichtung und die Verdunstungseinrichtung des Verschlusses derart gekoppelt sind, dass sie beide direkt oder indirekt mit der Flüssigkeit benetzbar sind.

5. Vorrichtung nach einem der vorhergehenden Ansprüche, bei der der zumindest eine Vorsprung zumindest eine Riffelung oder/und die Behälteröffnung (300, 503) eine zumindest teilweise korrespondierende Riffelung aufweist.

6. Vorrichtung nach einem der vorhergehenden Ansprüche, bei der sich der Innendurchmesser der Behälteröffnung (300, 503) zur Außenseite des Behälters (402, 502) hin verjüngt.

7. Vorrichtung nach einem der vorhergehenden Ansprüche, bei der die Dichteinrichtung einstückig mit dem Verschluss (100, 504, 506, 804, 904, 906) gebildet ist und der Verschluss und die Dichteinrichtung im Wesentlichen aus quellbarem Material bestehen.

8. Vorrichtung nach einem der vorhergehenden Ansprüche, weiter umfassend ein an dem Verschluss (100, 504, 506, 804, 904, 906) befestigtes, langgestrecktes Element (104, 508) und die Flüssigkeit über das Element (104, 508) in das quellbare Material des Verschlusses (100, 504, 506, 804, 904, 906) transportierbar ist.

9. Vorrichtung nach einem der vorhergehenden Ansprüche, bei der das Material, das der Verschluss (100, 504, 506, 804, 904, 906) zumindest teilweise umfasst, Holz oder quellbarer Kunststoff ist.

10. Vorrichtung nach einem der vorhergehenden Ansprüche, bei dem das langgestreckte Element (104, 508) in eine Bohrung im Verschluss (100, 504, 506, 804, 904, 906) einsetzbar ist und die Bohrung in der Ebene der Maserung des Holzes des Verschlusses (100, 504, 506, 804, 904, 906) liegt.

11. Vorrichtung nach einem der vorhergehenden Ansprüche, bei der zumindest ein weiteres Element (810) am Verschluss (100, 504, 506, 804, 904, 906) befestigbar ist.

12. Vorrichtung nach einem der vorhergehenden Ansprüche, bei der der Verschluss (100, 504, 506, 804, 904, 906, 1004) zumindest eine ebene äußere Fläche (1008) aufweist.

13. Vorrichtung nach einem der vorhergehenden Ansprüche, bei der der Behälter (402, 502, 1002) von einem Hüllkörper (510) umgeben ist, in dem die Form des Behälters ausgenommen ist, sodass der Behälter zumindest teilweise formschlüssig in den Hüllkörper (510) setzbar ist.

14. Vorrichtung nach einem der vorhergehenden Ansprüche, weiter umfassend eine Flüssigkeit, enthaltend einen Duftstoff und 25% bis 40% Gewichtsprozent Alkohohl.

15. Verschluss für eine Vorrichtung zum Verdunsten von Flüssigkeit mit einer Dichteinrichtung zum Verschließen einer Behälteröffnung eines zumindest teilweise mit Flüssigkeit befüllten Behälters (402, 502, 1002), wobei die Dichtung zumindest teilweise aus einem Material besteht, das durch Kontakt mit der Flüssigkeit quellbar ist, wobei durch das Quellen zumindest abschnittsweise eine kraftschlüssige Verbindung zwischen Behälteröffnung und Verschluss hergestellt oder verstärkt wird und der Verschluss weiterhin eine Verdunstungseinrichtungzum Verdunsten der Flüssigkeit umfasst.

## Claims

1. Device for evaporating liquid, wherein the device comprises:
a container (402, 502) for receiving a liquid (404), said container having at least one container opening (300, 503),
a closure (100, 504, 506, 804, 904, 906, 1100) with a sealing device for closing the container opening (300, 503, 803) and an evaporation device for evaporating the liquid, wherein the sealing device at least partially consists of a material which is swellable by contact with the liquid (404), wherein, as a result of the swelling, a frictional connection is at least partially produced or reinforced between the container opening (300, 503) and the closure (100, 504, 506, 804, 904, 906, 1100).

2. Device according to Claim 1, wherein the swellable material is obtained in such a manner that the liquid (404) is transportable through the swellable material.

3. Device according to Claim 1 or 2, in which the sealing device is formed by at least one projection on that side of the closure (100, 504, 506, 804, 904, 906) which is directed towards the container opening (300, 503), said projection being configured in such a manner that the projection rests in a frictional manner on the container opening (300, 503) on the outside and/or inside and/or upper side of the container (402, 502).

4. Device according to one of the preceding claims, in which the sealing device and the evaporation device of the closure are coupled in such a manner that they are both directly or indirectly wettable with the liquid.

5. Device according to one of the preceding claims, in which the at least one projection has at least one ribbing and/or the container opening (300, 503) has an at least partially corresponding ribbing.

6. Device according to one of the preceding claims, in which the internal diameter of the container opening (300, 503) tapers towards the outside of the container (402, 502).

7. Device according to one of the preceding claims, in which the sealing device is formed integrally with the closure (100, 504, 506, 804, 904, 906), and the closure and the sealing device substantially consist of swellable material.

8. Device according to one of the preceding claims, further comprising an elongate element (104, 508) which is fastened to the closure (100, 504, 506, 804, 904, 906), and the liquid is transportable into the swellable material of the closure (100, 504, 506, 804, 904, 906) via the element (104, 508).

9. Device according to one of the preceding claims, in which the material which at least partially surrounds the closure (100, 504, 506, 804, 904, 906) is wood or swellable plastic.

10. Device according to one of the preceding claims, in which the elongate element (104, 508) is insertable into a hole in the closure (100, 504, 506, 804, 904, 906), and the hole lies in the plane of the wood grain of the closure (100, 504, 506, 804, 904, 906).

11. Device according to one of the preceding claims, in which at least one further element (810) is fastenable to the closure (100, 504, 506, 804, 904, 906).

12. Device according to one of the preceding claims, in which the closure (100, 504, 506, 804, 904, 906, 1004) has at least one flat outer surface (1008).

13. Device according to one of the preceding claims, in which the container (402, 502, 1002) is surrounded by an enveloping body (510), in which the shape of the container is recessed, and therefore the container is fittable in an at least partially form-fitting manner into the enveloping body (510).

14. Device according to one of the preceding claims, further comprising a liquid containing a fragrance and 25% to 40% by weight of alcohol.

15. Closure for a device for evaporating liquid, with a sealing device for closing a container opening in a container (402, 502, 1002) which is at least partially filled with liquid, wherein the seal is at least partially composed of a material which is swellable by contact with the liquid, wherein, as a result of the swelling, a frictional connection is at least partially produced or reinforced between the container opening and the closure, and the closure furthermore comprises an evaporation device for evaporating the liquid.

## Revendications

1. Dispositif servant à évaporer un liquide, sachant que le dispositif comprend :
un récipient (402, 502) servant à recevoir un liquide (404), lequel récipient présente au moins une ouverture de récipient (300, 503),
un système de fermeture (100, 504, 506, 804, 904, 906, 1100) comprenant un système étanche servant à fermer l'ouverture de récipient (300, 503, 803) et comprenant un système d'évaporation servant à évaporer le liquide, sachant que le système étanche est constitué au moins en partie d'un matériau pouvant gonfler au contact du liquide (404), sachant que le gonflement permet d'établir ou de renforcer au moins par endroits un assemblage à force entre l'ouverture de récipient (300, 503) et le système de fermeture (100, 504, 506, 804, 904, 906, 1100).

2. Dispositif selon la revendication 1, sachant que le matériau pouvant gonfler présente des propriétés telles que le liquide (404) peut être transporté par le matériau pouvant gonfler.

3. Dispositif selon la revendication 1 ou 2, dans le cadre duquel le système étanche est formé par au moins une partie faisant saillie au niveau du côté, orienté en direction de l'ouverture de récipient (300, 503), du système de fermeture (100, 504, 506, 804, 904, 906), qui est configuré de manière à ce que la partie faisant saillie repose à force au niveau de l'ouverture de récipient (300, 503), sur le côté extérieur et/ou sur le côté intérieur et/ou sur le côté supérieur du récipient (402, 502).

4. Dispositif selon l'une quelconque des revendications précédentes, dans le cadre duquel le système étanche et le système d'évaporation du système de fermeture sont couplés de telle manière qu'ils peuvent être mouillés tous les deux directement ou indirectement par le liquide.

5. Dispositif selon l'une quelconque des revendications précédentes, dans le cadre duquel la partie faisant saillie au moins au nombre de une présente au moins une cannelure, et/ou en ce que l'ouverture de récipient (300, 503) présente une cannelure correspondante au moins en partie.

6. Dispositif selon l'une quelconque des revendications précédentes, dans le cadre duquel le diamètre intérieur de l'ouverture de récipient (300, 503) se rétrécit en direction du côté extérieur du récipient (402, 502).

7. Dispositif selon l'une quelconque des revendications précédentes, dans le cadre duquel le système étanche est formé d'un seul tenant avec le système de fermeture (100, 504, 506, 804, 904, 906), et en ce que le système de fermeture et le système étanche sont constitués essentiellement d'un matériau pouvant gonfler.

8. Dispositif selon l'une quelconque des revendications précédentes, comprenant en outre un élément (104, 508) allongé, fixé au niveau du système de fermeture (100, 504, 506, 804, 904, 906), le liquide pouvant être transporté par l'intermédiaire de l'élément (104, 508) dans le matériau pouvant gonfler du système de fermeture (100, 504, 506, 804, 904, 906).

9. Dispositif selon l'une quelconque des revendications précédentes, dans le cadre duquel le matériau que le système de fermeture (100, 504, 506, 804, 904, 906) comprend au moins en partie, est du bois ou un plastique pouvant gonfler.

10. Dispositif selon l'une quelconque des revendications précédentes, dans le cadre duquel l'élément (104, 508) allongé peut être introduit dans un alésage dans le système de fermeture (100, 504, 506, 804, 904, 906), et en ce que l'alésage se trouve dans le plan de la madrure du bois du système de fermeture (100, 504, 506, 804, 904, 906).

11. Dispositif selon l'une quelconque des revendications précédentes, dans le cadre duquel au moins un autre élément (810) peut être fixé au niveau du système de fermeture (100, 504, 506, 804, 904, 906).

12. Dispositif selon l'une quelconque des revendications précédentes, dans le cadre duquel le système de fermeture (100, 504, 506, 804, 904, 906, 1004) présente au moins une surface (1008) plane extérieure.

13. Dispositif selon l'une quelconque des revendications précédentes, dans le cadre duquel le récipient (402, 502, 1002) est entouré par un corps d'enveloppe (510), lequel reçoit le moule du récipient de sorte que le récipient peut être posé au moins en partie par complémentarité de forme dans le corps d'enveloppe (510).

14. Dispositif selon l'une quelconque des revendications précédentes, comprenant en outre un liquide qui contient une substance odorante et 25 % à 40 % de pourcentage de poids en alcool.

15. Système de fermeture destiné à un dispositif servant à évaporer un liquide comprenant un système étanche servant à fermer une ouverture de récipient d'un récipient (402, 502, 1002) rempli au moins en partie d'un liquide, sachant que le joint d'étanchéité est constitué au moins en partie d'un matériau qui peut gonfler au contact du liquide, sachant que le gonflement permet d'établir ou de renforcer au moins par endroits un assemblage à force entre l'ouverture de récipient et le système de fermeture et que le système de fermeture comprend en outre un système d'évaporation servant à évaporer le liquide.
